# EUROPEAN PATENT SPECIFICATION

(11) **EP 3 970 655 B1**
(45) Date of publication and mention of the grant of the patent: **03.05.2023**
(21) Application number: 19938023.9
(22) Date of filing: 30.08.2019
(51) Int. Cl.: A61C 7/00, A41D 13/11, A61C 19/04, A61C 7/08, G16H 40/40

(54) **SPLIT WIRELESS DETECTION MASK AND DETECTION METHOD THEREFOR**
GETEILTE DRAHTLOSE DETEKTIONSMASKE UND DETEKTIONSVERFAHREN DAFÜR
MASQUE DE DÉTECTION SANS FIL À FENTE ET PROCÉDÉ DE DÉTECTION CORRESPONDANT

(30) Priority: 16.07.2019 CN 201910641393
(43) Date of publication of application: 23.03.2022
(73) Proprietor: Chengheng (Shanghai) Health & Medical Devices Co., Ltd., China (Shanghai) Pilot Free Trade Zone Shanghai 200131 (CN)
(72) Inventor: YUE, Jiang, Shanghai 200131 (CN)
(74) Representative: Ipside
(86) International application number: PCT/CN2019/103653
(87) International publication number: WO 2021/007928

(56) References cited:
- WO-A1-2016/061279
- CN-A- 103 211 658
- CN-A- 106 031 660
- CN-A- 107 308 563
- CN-A- 107 468 499
- CN-A- 108 140 178
- CN-A- 108 308 757
- CN-A- 109 637 656
- CN-U- 206 365 935
- CN-U- 208 371 932
- KR-B1- 101 667 753
- US-A1- 2013 323 669
- US-A1- 2018 000 563

## Description

### Technical Field

The present invention relates to a wearable device technology, particularly to a split wireless detection mask and detection method therefore.

### Background of the Invention

The existing orthodontic treatment method is to use an appliance to a patient, such as a removable appliance, a functional appliance, a fixed appliance, a retainer, etc., by applying an appropriate "biological force" to the teeth, alveolar bones and jaw bones, so that they produce physiological movement. This method is slow in use process and long in effect taking time.

The patent with the application number 201811341718.1 proposed by the applicant proposes an accelerated orthodontic device and an accelerated orthodontic method, wherein a mask method is employed to correct the teeth of a patient. However, the wearing information of users cannot be collected, such as wearing length of time and the like. Due to the fact that the information feedback is not enough in time and the expression of some patients is unclear, the treatment method implemented and treatment results are biased, the accurate collection of data of the whole treatment method cannot be realized, the treatment regimen cannot be accurately improved, thus adverse to the accurate and customized correction of the patient.

A patent application No. US 2013/323669 A1 discloses a bite plate allowing for contact with occlusal and facial and/or lingual maxillary and mandibular teeth is coupled to an extraoral housing containing a rechargeable battery coupled to a vibrator coupled to a processor coupled to data and charging port or ports. The housing is at least water resistant, and a hatch allows access to the data and charging port or ports only. The device is fitted with a very smooth and quiet vibrator, with minimal variance on operating specifications.

A patent application No. US 2018/000563 A1 discloses an intraoral appliances with sensing for the detection of placement of dental aligners in patient mouth on teeth for indication of wearing compliance. Described herein are apparatuses and methods for detecting wearing, including compliance, and for reliably transferring data, by wired or wireless direct or indirect communication of electronic compliance information to a smartphone. Also described herein are dental appliances that can detect physiological parameters related to respiration and sleep. Also described herein are aligner cases that enable NFC communication with electronic compliance indicator (ECI) devices and Bluetooth communication with smartphones.

A patent application No. WO 2016/061279 A1 discloses a dental usage monitoring system having a dental appliance, a sensor unit having at least one sensor attached to the dental appliance, a power supply attached to the dental appliance, and an analyzer. The at least one sensor attached to the dental appliance is configured to collect data related to usage of the dental appliance. An analyzer in communication with the sensor and is configured to determine usage of the dental appliance based upon the collected data. The dental usage monitoring system can include a base module configured to receive collected data, transmit data to the analyzer, and recharge the sensor unit. The base module can be further configured to calibrate the collected data. The sensor unit can be embedded in the dental appliance or coupled to the dental appliance. The sensor unit may be encased in a protective coating.

### Summary of the Invention

The problem to be solved by the present invention is to compensate for the above-mentioned drawbacks of the prior art, and to provide a split wireless detection mask and detection method therefor that can detect wearing information of a user, and transmit to a cloud server at an appropriate time.

The invention is set out in the appended set of claims.

The technical problem of the invention can be solved by the following technical scheme:
A split wireless detection mask, comprising a mask and a base that are split and may be connected to each other. The mask is being worn on a human body and used for collecting wearing information of the human body, and when the mask is connected to the base, the base may charge the mask, the mask transmits the collected wearing information to the base, and the base transmits the information to a cloud server.

Further, the mask comprises a control unit, a detection unit, a power supply unit and a detection information transmitting unit. The detection unit, the power supply unit and the detection information transmitting unit are electrically connected to the control unit respectively, the power supply unit charges the mask, and the wearing information is collected by the detection unit.

Further, the base comprises a wireless transmission unit, a power source unit and a detection information receiving unit. The power source unit is connected to an external power source, the wireless transmission unit, the power source unit and the detection information receiving unit are electrically connected, and the detection information receiving unit is used for receiving data sent by the detection information transmitting unit.

Further, the wearing information comprises information about whether a mask is being worn on a human body, information about length of time of the mask being worn and other information about the mask being worn.

Further, if the detection unit detects that a human body wears the mask, the control unit starts timing, and when the detection unit detects that the human body removes the mask, the control unit stops timing so as to detect the information about the length of time of the mask being worn on the human body.

Further, other information about the mask being worn comprises information used to characterize the correction effect of the mask as an orthodontic appliance, including a wearing posture, a stress between the mask and teeth.

Further, connection of the mask to the base is an electrical connection, and information between the mask and the base is transmitted wired or wireless; and data transmission is realized between the base and the cloud server through a wireless transmission unit.

Further, either a jack or a magnetic connector is used for connecting the mask and the base.

Further, the power supply unit further comprises a charging unit.

Further, the detection unit is a capacitive touch sensing circuit.

Further, the capacitive touch sensing circuit adopts a RC acquisition principle, and the capacitive touch sensing circuit comprises a plurality of resistors, a plurality of capacitors and a plurality of reserved contacts, and the reserved contacts comprise a conductive metal sheet for detecting whether a human body is connected to the detection unit.

Further, when a human body contacts the reserved contacts causing the capacitive touch sensing circuit to conduct, it is equivalent to connecting a capacitor, applying a periodic charging voltage to the capacitive touch sensing circuit, detecting the voltage across a capacitor, and indicating that the human body has contacted the detection unit when the time required for the voltage across the capacitor to reach a threshold is extended.

Further, the control unit comprises a controller, and the model of the controller is either STM32F103C8T6 or ATmega8.

Further, the control unit comprises a timing unit, and the timing unit sets a fixed time, and continuously transmits a signal to the control unit periodically at the fixed time. The control unit detects whether there is any human body contact in the detection unit, and if the human body is already in contact, the control unit takes a transmission signal of the timing unit at the moment when the human body is already in contact as timing start, and when the control unit receives no signal of the human body contact in the detection unit, a transmission signal of the timing unit at this point is considered as timing stop.

Further, the control unit comprises a storage unit, the storage unit stores information detected by the detection unit, and the storage unit is a non-volatile storage unit.

Further, a transmission method of the wireless transmission unit is one or more of GSM, WiFi or Bluetooth.

The present invention further provides a method for collecting length of time being worn on a human body utilizing the split wireless detection mask as mentioned above. A detection unit in the mask is utilized to detect whether a human body wears the mask, wearing length of time or other wearing information; if the human body wears the mask, the control unit starts timing and if the human body removes the mask, the control unit stops timing, and the detection unit transmits and stores the information about the wearing length of time and other wearing information to the control unit; when the mask is fixedly connected to the base, data in the control unit is transmitted to the base, then to a cloud server through a wireless transmission unit in the base, achieving real-time collection and transmission of wearing information of the human body.

Compared with the prior art, the beneficial effects of the present invention are that:
The present invention adopts a structural design of separating the data collection part and the data transmission part, and installing the data collection part in the mask, which may control the data collection part to a small volume, realize the miniaturization of the collection circuit, and therefore realize the lightweight installation of the data collection part in the mask. Meanwhile, the use of the power supply unit inside the mask can realize long-time data collection, and the collected data is stored in the internal non-volatile storage unit, for example, EEPROM or Flash Memory, so that the stability of data storage is guaranteed.

In the present invention, the data collection part and the data transmission part can be used for charging and data transmission through a jack or a magnetic connector and the like to facilitate connection; when the mask at the data collection on part is idle, the mask may be connected to the base for charging; the base is provided with a power source unit and a wireless transmission unit; data in the mask may be transmitted to a cloud server through the base while charging the mask, so that uploading of the data is realized, and physicians may obtain wearing information about patients in time.

By adopting the split wireless detection mask provided by the present invention to collect wearing information of a user, such as wearing length of time and the like, deviations of data in correction regimes caused by wearing problems of the user may be reduced, the correction efficiency may be improved, the correction period may be shortened, and the accuracy of data may be increased. Meanwhile, in the present invention, by real-time collection of the information about the length time being worn on the user and sending back to the cloud server at the appropriate time, data collection is realized more timely when compared with a return visit of a patient, the accuracy of the data is increased, to provide accurate data for physicians during treatment, which may improve the entire treatment process in time, as well as the trust between patients and physicians.

### Brief description of the Drawings

FIG. 1 is a schematic diagram of a detection unit according to the present invention;
FIG. 2 is a graph of charging and discharge time of the detection unit according to the present invention when not in contact with a human body;
FIG. 3 is a control diagram of the charging and discharge time of the detection unit according to the present invention before and after in contact with the human body;
FIG. 4 is an actual circuit diagram of the detection unit according to the present invention;
FIG. 5 is a block diagram of a split wireless detection mask according to the present invention.

### Detailed Description of Embodiments

Hereinafter, the present invention will be described further with reference to the preferred embodiments and with reference to the accompanying drawings.

In addition, the various components on the drawing are enlarged (thick) or reduced (thin) for the convenience of understanding, but this practice is not intended to limit the scope of protection of the present invention.

Words importing the singular number include the plural and vice versa.

In the description of the embodiments of the present invention, it should be noted that, the orientation or positional relationship indicated by the terms "upper," "lower," "inner," "outer," etc., is based on the orientation or positional relationship shown in the figures, or the orientation or positional relationship conventionally placed when the product is used, is merely for the convenience of describing the present invention and the simplified description, and is not intended to indicate or imply that the indicated device or element must have a particular orientation, constructed and operated in a particular orientation, and therefore not to be construed as a limitation of the present invention. In addition, in the description of the present invention, in order to distinguish different cells, the present description uses words of first, second, etc., but these are not limited by the order of manufacture, nor are it to be understood as indicating or implying relative importance, which may differ from the following detailed description of the invention and the claims.

The words in the description are used for the purpose of illustrating the embodiments of the present invention and are not intended to be limiting the present invention. It should also be noted that, unless expressly specified and defined otherwise, the terms "set," "attached," and "connected," are to be construed broadly, for example, may be fixedly connected, may be detachably connected, or integrally connected; may be mechanically connected, may be directly attached, may also be indirectly connected by an intermediate medium, may be internal communication of the two elements. It will be understood by those skilled in the art that the foregoing is a specific meaning in the present invention.

The present invention provides a split wireless detection mask, comprising a data collection part and a data transmission part which are split and may be connected to each other, and the data collection part is installed in the mask, and the data transmission part is installed in the base, a detection unit is arranged in the mask to collect the wearing information of users, such as information about whether a human body wears the mask, information about length of time of the mask being worn and other information about the mask being worn, and store data in a control unit in the mask. When the mask is idle, the mask may be connected to the base, the base is internally provided with a power source unit and a wireless transmission unit, the mask may be charged through the base, meanwhile, the mask may transmit the collected wearing information data to the base, and then transmit to a cloud server through a wireless transmission unit in the base to complete data collection and data transmission of the mask.

Because the mask in the present invention is used in the field of human orthodontics, other information about the mask being worn comprises information used to characterize the correction effect of the mask as an orthodontic appliance, including a wearing posture, a stress between the mask and teeth and the like. In the embodiment, the mask is used for collecting information about length of time being worn on a human body.

The mask comprises a control unit, a detection unit, a power supply unit and a detection information transmitting unit; the detection unit, the power supply unit and the detection information transmitting unit are electrically connected to the control unit respectively; the power supply unit is electrically connected to the detection information transmitting unit; the power supply unit supplies power to the entire mask; the control unit is used for processing the information collected by the detection unit; the detection unit is embedded in the mask and realizes the detection of whether a human body wears the mask or not by contacting the skin of the human body, and when the mask is worn on an inanimate object, the detection unit may identify whether it is worn by a human body due to the fact that the skin of the human body is not in contact.

In the embodiment of the invention, the control unit comprises a controller, and the model of the controller is either STM32F103C8T6 or ATmega8.

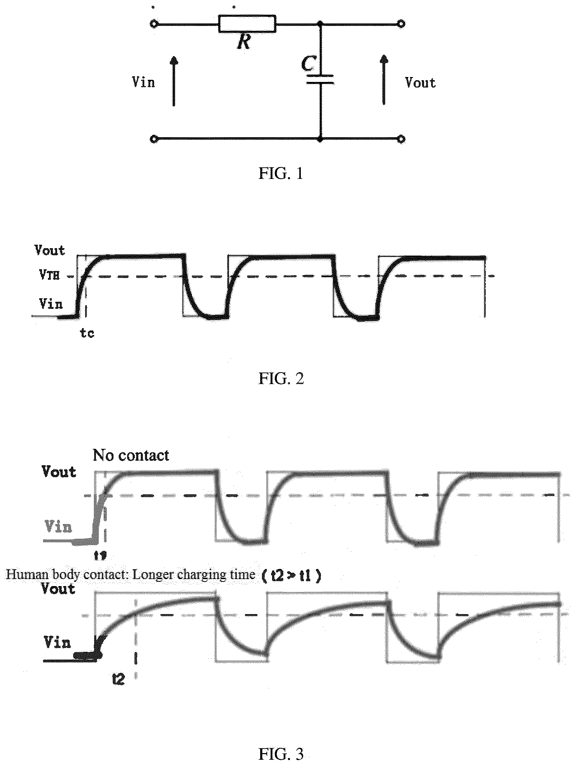

Specifically, the detection principle of the detection unit is a phase shift circuit, namely a RC acquisition circuit. As shown in FIG. 1, a capacitor and a resistor are arranged in the RC acquisition circuit. Meanwhile, reserved contacts are arranged in the circuit. When a human body contacts the reserved contacts, contact of the human body to the reserved contacts of the circuit is equivalent to connecting a capacitor, so that circuit parameters are changed. At this time, whether a human body is put in circuit or not may be detected by measuring the charging and discharge time of the capacitor in the RC circuit.

As shown in FIG. 1, under normal circumstances, applying a periodic charging voltage Vin to the RC circuit and measuring the Vout terminal to may result in a graph of the charging and discharge time of the capacitor as shown in FIG. 2, wherein the horizontal axis represents time, the longitudinal axis represents the voltage across the capacitor, and whether a human body is in contact or not can be judged by detecting the difference between the charging start time of the capacitor to the time tc of the Vout terminal to reach a certain threshold Vth. As shown in FIG. 3, when a human body contacts a circuit, the capacitor charging time becomes longer, that is, t2 > t1.

In the present invention, the power supply unit is a rechargeable lithium battery, the voltage thereof is 3.7 V, the model number is 401235, and the threshold Vth measured at the VOUT terminal is 2.5 V.

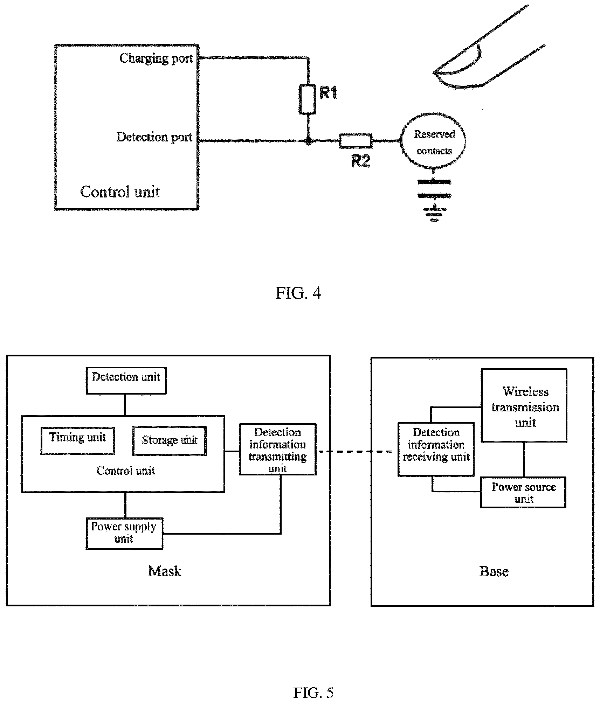

The circuit diagram of the detection unit in the mask is as shown in FIG. 4, the control unit is connected with two ports, i.e. a charging port and a detection port, and the charging port and the detection port are respectively connected to the detection unit, the charging port charges the capacitor, and the detection port measures the voltage across the capacitor, wherein the resistance value of R1 is 1 megaohm, used for controlling the charging time range, and the resistance value of R2 is 10 kilo ohms, used for reducing noise interference.

The detection unit is provided with reserved contacts for contact with a human body. These reserved contacts are a conductive metal sheet, such as a stainless steel or copper sheet and the like, and the human body is in contact with the reserved contacts so as to be put in a circuit of the detection unit.

When the charging port is set, the detection port detects whether a threshold of a trigger voltage is reached. When the human body is in contact, the detection port detects the threshold of reaching the trigger voltage, and at this time, the control unit detects that tc changes, indicating that the human body is in contact into the detection unit, thereby realizing the detection of human body contact.

The detection unit may also adopt a capacitive touch sensing circuit or a capacitive touch sensing chip to realize detection, the principle thereof is the same as the RC circuit, and transmit the detection result to the control unit.

The mask further comprises a timing unit and a storage unit arranged on the control unit. The timing unit is provided with a fixed time, so that the signal can be transmitted to the control unit in a timed manner. The control unit re-checks whether voltage on the detection port in the detection unit reaches a threshold or not, if the threshold is reached, indicating that the human body is in contact, the control unit takes the signal transmitted by the timing unit at this point as timing start. When the control unit checks that the voltage on the detection port in the detection unit does not reach the threshold, indicating that the human body is not in contact, the control unit takes the signal transmitted by the timing unit at this point as timing stop, calculates the time from reaching the threshold to the time not reaching the threshold, that is, the length of time of the human body in contact into the detection unit, so that the length of time being worn on a user is detected, and the data on length of time of being worn is transmitted to the storage unit in the control unit, and saved.

The storage unit is a non-volatile storage unit, such as an Electrically Erasable Programmable Read-Only Memory (EEPROM) or a Flash Memory.

Since the time interval set by the timing unit is the same, the accuracy of the wearing time measurement is the time set by the timing unit. The timing unit transmits a signal to the control unit every set time, the detection unit detects whether the human body wears the mask, and transmits the signal to the control unit, so that the actual length of time being worn on users may be accurately detected, and the detection data may be stored in the storage unit.

The mask comprises a power supply unit electrically connected to the control unit, and a detection information transmitting unit. The power supply unit supplies power to the entire mask, the detection information transmitting unit is electrically connected to the power supply unit and the control unit respectively, and data in the storage unit is transmitted out of the mask through the detection information transmitting unit.

The base comprises a power source unit, a wireless transmission unit and a detection information receiving unit. The wireless transmission unit and the detection information receiving unit are electrically connected to the power source unit respectively. The power source unit is connected to an external power socket to supply power source to the base, and the wireless transmission unit and the detection information receiving unit are electrically connected.

A transmission method of the wireless transmission unit is one or more of GSM, WiFi or Bluetooth.

The detection information receiving unit and the detection information transmitting unit may be flexibly connected using, for example, a jack or a magnetic connector.

When the mask is idle, the connection between the mask and the base may be realized through the connection between the detection information transmitting unit and the detection information receiving unit. The power source unit may charge the power supply unit in the mask through two interfaces, the storage unit in the mask may transmit data to the wireless transmission unit through two interfaces, the wireless transmission unit transmits data to a cloud server to complete data collection and transmission. The data may facilitate an orthodontist to timely master the wearing information of a patient, and avoid affecting the treatment of the orthodontist due to unclear expression of the patient or too long return visit time.

The present invention further provides a method for detecting the above-mentioned length of time of the mask being worn. A detection unit in the mask is utilized to detect whether a human body wears the mask. If the human body wears the mask, the detection unit transmits a signal to a control unit in the mask, the control unit starts timing; if the human body removes the mask, the control unit stops timing, and the control unit detects the information about the length of time being worn on the human body, and stores the information in the control unit; when the detection information transmitting unit and the detection information receiving unit are fixly connected, data in the control unit is transmitted to the base, then to a cloud server through a wireless transmission unit, achieving real-time collection and transmission of the information about the length of time being worn on the human body. Detection of other wearing information by the mask may obtain related information of orthodontic effects by arranging a pressure sensor, an ultrasonic sensor or an image sensor according to needs in the detection unit of the mask.

The above has been described in detail with respect to the specific embodiments of the present invention. It will be apparent to those skilled in the art that various modifications and adaptations may be made to the present invention without departing from the principles of the invention, which are also intended to be within the scope of the appended claims.

## Claims

1. A split wireless detection mask
comprising a mask and a base which are split and may be connected to each other, the mask is adapted to be worn on a human body and used for collecting wearing information of the human body; when the mask is connected to the base, the base is adapted to charge the mask, the mask transmits the collected wearing information to the base, and the base transmits the information to a cloud server; the mask comprises a control unit, a detection unit, a power supply unit and a detection information transmitting unit; wherein the detection unit, the power supply unit and the detection information transmitting unit are electrically connected to the control unit respectively, the power supply unit charges the mask, and the wearing information is collected by the detection unit;
wherein the detection unit is a capacitive touch sensing circuit, wherein, the capacitive touch sensing circuit is configured to adopt a RC acquisition principle, and comprises a plurality of resistors, a plurality of capacitors and a plurality of reserved contacts, and the reserved contacts comprise a conductive metal sheet for detecting whether the human body is connected to the detection unit; a pressure sensor, an ultrasonic sensor or an image sensor are arranged in the detection unit;
the wearing information comprises information about whether the human body is wearing a mask, information about length of time of the mask being worn and other information of the mask being worn; wherein the other information about the mask being worn comprises information used to characterize the correction effect of the mask as an orthodontic appliance, including a wearing posture, a stress between the mask and teeth;
the other information about the mask being worn is obtained through the pressure sensor, the ultrasonic sensor or the image sensor in the detection unit of the mask.

2. The split wireless detection mask according to claim 1, wherein, the base comprises a wireless transmission unit, a power source unit and a detection information receiving unit; wherein the power source unit is connected to an external power source, the wireless transmission unit, the power source unit and the detection information receiving unit are electrically connected, and the detection information receiving unit is used for receiving data sent by the detection information transmitting unit.

3. The split wireless detection mask according to claim 1, wherein, if the detection unit detects that the human body wears the mask, the control unit starts timing, and when the detection unit detects that the human body removes the mask, the control unit stops timing so as to detect the information about the length of time of the mask being worn on the human body.

4. The split wireless detection mask according to one of claims 1-3, wherein, the connection of the mask to the base is an electrical connection, and information between the mask and the base is transmitted wired or wireless; the base and the cloud server achieve data transmission through a wireless transmission unit.

5. The split wireless detection mask according to one of claims 1-3, wherein, either a jack or a magnetic connector is used for connecting the mask and the base.

6. The split wireless detection mask according to claim 1, wherein, the power supply unit further comprises a charging unit.

7. The split wireless detection mask according to claim 1, wherein, when the human body contacts the reserved contacts causing a capacitive touch sensing circuit to conduct, it is equivalent to connecting a capacitor, applying a periodic charging voltage to the capacitive touch sensing circuit, detecting the voltage across a capacitor, and indicating that the human body has contacted the detection unit when the time required for the voltage across the capacitor to reach a threshold is extended.

8. The split wireless detection mask according to claims 1-3, wherein, the control unit comprises a timing unit, and the timing unit sets a fixed time, and continuously transmits a signal to the control unit periodically at the fixed time; wherein the control unit detects whether there is human body contact in the detection unit, and if the human body is already in contact, the control unit starts timing by taking a transmission signal of the timing unit at the moment when the human body is already in contact, and when the control unit receives no signal of the human body contact in the detection unit, the transmission signal of the timing unit at this point is considered as timing stop.

9. The split wireless detection mask according to claim 1, wherein, the control unit comprises a storage unit, the storage unit stores information detected by the detection unit, and the storage unit is a non-volatile storage unit.

10. A method for collecting information about wearing by a human body utilizing the split wireless detection mask according to any one of claims 1-9, wherein, the detection unit in the mask is utilized to detect whether the human body is wearing the mask, wearing length of time or other wearing information; when the human body wears the mask, the control unit starts timing and when the human body removes the mask, the control unit stops timing, and the detection unit transmits and stores the information about the wearing length of time and other wearing information to the control unit; when the mask is fixedly connected to the base, data in the control unit is transmitted to the base, then to the cloud server through a wireless transmission unit in the base, achieving real-time collection and transmission of wearing information of the human body.

## Patentansprüche

1. Geteilte drahtlose Detektionsmaske, umfassend eine Maske und eine Basis, die geteilt sind und miteinander verbunden werden können, wobei die Maske ausgelegt ist, um an einem menschlichen Körper getragen zu werden, und zum Sammeln von Trageinformationen des menschlichen Körpers verwendet wird, wenn die Maske mit der Basis verbunden ist, wobei die Basis ausgelegt ist, um die Maske aufzuladen, die Maske die gesammelten Trageinformationen an die Basis überträgt und die Basis die Informationen an einen Cloud-Server überträgt; die Maske eine Steuereinheit, eine Detektionseinheit, eine Stromversorgungseinheit und eine Detektionsinformations-Sendeeinheit umfasst; wobei die Detektionseinheit, die Stromversorgungseinheit und die Detektionsinformations-Übertragungseinheit jeweils elektrisch mit der Steuereinheit verbunden sind, die Stromversorgungseinheit die Maske auflädt und die Trageinformationen von der Detektionseinheit gesammelt werden;
wobei die Detektionseinheit eine kapazitive Berührungsabtastschaltung ist, wobei die kapazitive Berührungsabtastschaltung so konfiguriert, dass sie das RC-Erfassungsprinzip übernimmt, und eine Vielzahl von Widerständen, eine Vielzahl von Kondensatoren und eine Vielzahl von reservierten Kontakten umfasst, und die reservierten Kontakte ein leitendes Metallblech zum Detektieren umfassen, ob der menschliche Körper mit der Detektionseinheit ist; wobei in der Detektionseinheit ein Drucksensor, ein Ultraschallsensor oder ein Bildsensor angeordnet sind;
wobei die Trageinformationen Informationen darüber, ob der menschliche Körper eine Maske trägt, Informationen über die Zeitdauer, während der die Maske getragen wird, und andere Informationen über die getragene Maske umfassen; wobei die anderen Informationen über die getragene Maske Informationen umfassen, die verwendet werden, um die Korrekturwirkung der Maske als kieferorthopädisches Gerät zu charakterisieren, einschließlich einer Tragehaltung und einer Spannung zwischen der Maske und den Zähnen;
wobei die anderen Informationen über die getragene Maske durch den Drucksensor, den Ultraschallsensor oder den Bildsensor in der Detektionseinheit der Maske erhalten werden.

2. Geteilte drahtlose Detektionsmaske nach Anspruch 1, wobei die Basis eine drahtlose Übertragungseinheit, eine Stromquelleneinheit und eine Detektionsinformations-Empfangseinheit umfasst; wobei die Stromquelleneinheit mit einer externen Stromquelle verbunden ist, die drahtlose Übertragungseinheit, die Stromquelleneinheit und die Detektionsinformations-Empfangseinheit elektrisch miteinander verbunden sind und die Detektionsinformations-Empfangseinheit zum Empfangen von Daten verwendet wird, die von der Detektionsinformations-Übertragungseinheit gesendet werden.

3. Geteilte drahtlose Detektionsmaske nach Anspruch 1, wobei, wenn die Detektionseinheit detektiert, dass der menschliche Körper die Maske trägt, die Steuereinheit die Zeitmessung startet, und wenn die Detektionseinheit detektiert, dass der menschliche Körper die Maske entfernt, die Steuereinheit die Zeitmessung stoppt, um die Informationen über die Zeitdauer zu detektieren, während der die Maske am menschlichen Körper getragen wird.

4. Geteilte drahtlose Detektionsmaske nach einem der Ansprüche 1-3, wobei die Verbindung der Maske mit der Basis eine elektrische Verbindung ist und Informationen zwischen der Maske und der Basis drahtgebunden oder drahtlos übertragen werden; die Basis und der Cloud-Server eine Datenübertragung durch eine drahtlose Übertragungseinheit erzielen.

5. Geteilte drahtlose Detektionsmaske nach einem der Ansprüche 1-3, wobei entweder eine Buchse oder ein magnetischer Verbinder zum Verbinden der Maske und der Basis verwendet wird.

6. Geteilte drahtlose Detektionsmaske nach Anspruch 1, wobei die Stromversorgungseinheit ferner eine Ladeeinheit umfasst.

7. Geteilte drahtlose Detektionsmaske nach Anspruch 1, wobei, wenn der menschliche Körper die reservierten Kontakte berührt, was bewirkt, dass eine kapazitive Berührungsabtastschaltung leitet, dies äquivalent ist zum Anschließen eines Kondensators, Anlegen einer periodischen Ladespannung an die kapazitive Berührungsabtastschaltung, Detektieren der Spannung über einem Kondensator und Anzeigen, dass der menschliche Körper die Detektionseinheit kontaktiert hat, wenn die Zeit verlängert wird, die erforderlich ist, damit die Spannung über dem Kondensator einen Schwellenwert erreicht.

8. Geteilte drahtlose Detektionsmaske nach den Ansprüchen 1-3, wobei die Steuereinheit eine Zeitsteuerungseinheit umfasst, und die Zeitsteuerungseinheit einen festgelegten Zeitpunkt einstellt und kontinuierlich periodisch zum festgelegten Zeitpunkt ein Signal an die Steuereinheit überträgt; wobei die Steuereinheit detektiert, ob ein menschlicher Körperkontakt in der Detektionseinheit besteht, und wenn der menschliche Körper bereits in Kontakt steht, startet die Steuereinheit die Zeitmessung, indem sie ein Übertragungssignal der Zeitsteuerungseinheit in dem Moment nimmt, in dem der menschliche Körper bereits in Kontakt ist, und wenn die Steuereinheit kein Signal des menschlichen Körperkontakts in der Detektionseinheit empfängt, wird das Übertragungssignal der Zeitsteuerungseinheit an diesem Punkt als Zeitsteuerungsstopp betrachtet.

9. Geteilte drahtlose Detektionsmaske nach Anspruch 1, wobei die Steuereinheit eine Speichereinheit umfasst, die Speichereinheit von der Detektionseinheit detektierte Informationen speichert und die Speichereinheit eine nichtflüchtige Speichereinheit ist.

10. Verfahren zum Sammeln von Informationen über das Tragen durch einen menschlichen Körper der geteilten drahtlosen Detektionsmaske nach einem der Ansprüche 1-9, wobei die Detektionseinheit in der Maske verwendet wird, um zu detektieren, ob der menschliche Körper die Maske trägt, sowie die Tragedauer oder andere Trageinformationen; wenn der menschliche Körper die Maske trägt, startet die Steuereinheit mit der Zeitmessung, und wenn der menschliche Körper die Maske entfernt, stoppt die Steuereinheit die Zeitmessung, und die Detektionseinheit überträgt die Informationen über die Tragedauer und andere Trageinformationen an die Steuereinheit und speichert sie; wenn die Maske fest mit der Basis verbunden ist, werden Daten in der Steuereinheit an die Basis und dann über eine drahtlose Übertragungseinheit in der Basis an den Cloud-Server übertragen, wodurch eine Sammlung und Übertragung von Trageinformationen des menschlichen Körpers in Echtzeit erreicht wird.

## Revendications

1. Masque de détection sans fil à fente comprenant un masque et une base qui ont une fente et qui peuvent être raccordés l'un à l'autre, le masque est adapté à être porté sur un corps humain et utilisé pour collecter des informations de port du corps humain ; lorsque le masque est raccordé à la base, la base est adaptée à charger le masque, le masque transmet les informations de port collectées à la base, et la base transmet les informations à un serveur en nuage ; le masque comprend une unité de commande, une unité de détection, une unité de source d'énergie et une unité de transmission d'informations de détection ; dans lequel l'unité de détection, l'unité de source d'énergie et l'unité de transmission d'informations de détection sont électriquement raccordées à l'unité de commande, respectivement, l'unité de source d'énergie charge le masque, et les informations de port sont collectées par l'unité de détection ;
dans lequel l'unité de détection est un circuit de détection tactile capacitif, dans lequel, le circuit de détection tactile capacitif est configuré pour adopter un principe d'acquisition RC, et comprend une pluralité de résistances, une pluralité de condensateurs et une pluralité de contacts réservés, et les contacts réservés comprennent une feuille de métal conducteur pour détecter si le corps humain est raccordé à l'unité de détection ; un capteur de pression, un capteur ultrasonore ou un capteur d'image sont agencés dans l'unité de détection ; les informations de port comprennent des informations relatives au port ou non d'un masque par le corps humain, des informations relatives à la durée de port du masque et d'autres informations relatives au masque étant porté ; dans lequel les autres informations relatives au masque étant porté comprennent des informations utilisées pour caractériser l'effet de correction du masque en tant qu'appareil orthodontique, y compris une posture de port, une contrainte entre le masque et les dents ;
les autres informations relatives au masque étant porté sont obtenues par l'intermédiaire du capteur de pression, du capteur ultrasonore ou du capteur d'image dans l'unité de détection du masque.

2. Masque de détection sans fil à fente selon la revendication 1, dans lequel la base comprend une unité de transmission sans fil, une unité de source d'énergie et une unité de réception d'informations de détection ; dans lequel l'unité de source d'énergie est raccordée à une source d'énergie externe, l'unité de transmission sans fil, l'unité de source d'énergie et l'unité de réception d'informations de détection sont électriquement raccordées, et l'unité de réception d'informations de détection est utilisée pour recevoir des données envoyées par l'unité de transmission d'informations de détection.

3. Masque de détection sans fil à fente selon la revendication 1, dans lequel si l'unité de détection détecte que le corps humain porte le masque, l'unité de commande commence à chronométrer, et lorsque l'unité de détection détecte que le corps humain retire le masque, l'unité de commande arrête de chronométrer de façon à détecter les informations relatives à la durée de port du masque sur le corps humain.

4. Masque de détection sans fil à fente selon l'une quelconque des revendications 1 à 3, dans lequel le raccordement du masque à la base est un raccordement électrique, et les informations entre le masque et la base sont transmises avec ou sans fil ; la base et le serveur en nuage parviennent à une transmission de données par l'intermédiaire d'une unité de transmission sans fil.

5. Masque de détection sans fil à fente selon l'une quelconque des revendications 1 à 3, dans lequel un jack ou un raccord magnétique est utilisé pour raccorder le masque et la base.

6. Masque de détection sans fil à fente selon la revendication 1, dans lequel, l'unité de source d'énergie comprend en outre une unité de charge.

7. Masque de détection sans fil à fente selon la revendication 1, dans lequel, lorsque le corps humain contacte les contacts réservés provoquant la conduction par un circuit de détection tactile capacitif, cela équivaut à raccorder un condensateur, appliquer une tension de charge périodique au circuit de détection tactile capacitif, détecter la tension à travers un condensateur, et indiquer que le corps humain a contacté l'unité de détection lorsque le temps requis pour que la tension à travers le condensateur atteigne un seuil est prolongé.

8. Masque de détection sans fil à fente selon l'une quelconque des revendications 1 à 3, dans lequel l'unité de commande comprend une unité de chronométrage, et l'unité de chronométrage définit une durée fixe, et transmet en continu un signal à l'unité de commande périodiquement à la durée fixe ; dans lequel l'unité de commande détecte s'il y a un contact avec le corps humain dans l'unité de détection, et si le corps humain est déjà en contact, l'unité de commande commence à chronométrer en prenant un signal de transmission de l'unité de chronométrage au moment où le corps humain est déjà en contact, et lorsque l'unité de commande ne reçoit pas de signal du contact avec le corps humain dans l'unité de détection, le signal de transmission de l'unité de chronométrage à ce point est considéré comme un arrêt de chronométrage.

9. Masque de détection sans fil à fente selon la revendication 1, dans lequel, l'unité de commande comprend une unité de stockage, l'unité de stockage stocke des informations détectées par l'unité de détection, et l'unité de stockage est une unité de stockage non volatile.

10. Procédé de collecte d'informations relatives au port par un corps humain utilisant le masque de détection sans fil à fente selon l'une quelconque des revendications 1 à 9, dans lequel, l'unité de détection dans le masque est utilisée pour détecter si le corps humain porte le masque, la durée du port ou autres informations relatives au port ; lorsque le corps humain porte le masque, l'unité de commande commence à chronométrer et lorsque le corps humain retire le masque, l'unité de commande arrête de chronométrer, et l'unité de détection transmet et stocke les informations relatives à la durée de port et autres informations de port à l'unité de commande ; lorsque le masque est raccordé de manière fixe à la base, les données dans l'unité de commande sont transmises à la base, puis au serveur en nuage par l'intermédiaire d'une unité de transmission sans fil dans la base, permettant une collecte et une transmission en temps réel des informations de port du corps humain.
